# EUROPEAN PATENT APPLICATION

(11) **EP 3 320 927 A1**
(43) Date of publication of application: **16.05.2018**
(21) Application number: 16875679.9
(22) Date of filing: 14.12.2016
(51) Int. Cl.: A61M 1/00, A61M 16/04

(54) **AIRWAY DEVICE AND BODY FLUID SUCTION TUBE AS WELL AS METHOD FOR USING SAME**

(30) Priority: 18.12.2015 JP 2015247146
(71) Applicant: Tokuso Giken Co., Ltd., Usa-shi, Oita 879-0232 (JP)
(72) Inventor: TOKUNAGA Shuichi, Usa-shi Oita 879-0232 (JP)
(74) Representative: Schäfer, Matthias W.
(86) International application number: PCT/JP2016/087218
(87) International publication number: WO 2017/104702

(57) **Abstract**

An airway device includes a body fluid suction tube; a tracheal insertion tube to be passed through a trachea of a patient for sucking in a body fluid in the trachea of the patient; and an adaptor disposed at a base end portion of the tracheal insertion tube to be freely rotatable. The body fluid suction tube is arranged so that an opening portion thereof is situated inside relative to an opening portion of the tracheal insertion tube. The body fluid suction tube is arranged so that only the distal end portion thereof is urged against an inner surface of the tracheal insertion tube through an elastic force. The body fluid suction tube is arranged so that a lower side of an outer surface thereof is separated and is not closely contact with an upper side of the inner surface of the tracheal insertion tube.

## Description

### TECHNICAL FIELD

The present invention relates to an airway device in which a body fluid suction tube is inserted into a tracheal insertion tube to be passed through a trachea of a patient, so that the body fluid suction tube can suck in a body fluid such as phlegm and saliva in the trachea of the patient. Further, the present invention relates to a body fluid suction tube is inserted into a tracheal insertion tube to be passed through a trachea of a patient, so that the body fluid suction tube can suck in a body fluid such as phlegm and saliva in the trachea of the patient. Further, the present invention relates to a method of using the airway device and the body fluid suction tube.

### BACKGROUND ART

Conventionally, when an artificial respirator and the like is operated, a conventional tracheal insertion tube such as a tracheal cannula and an insertion tube is inserted into a trachea of a patient, so that an air passage of the patient is secured.

When the conventional tracheal insertion tube is inserted into the trachea of the patient, it is difficult for the patient to discharge a body fluid such as phlegm and saliva in the trachea with own power. In order to discharge the body fluid, a suction catheter such as a conventional body fluid suction tube is inserted into the conventional tracheal insertion tube. Then, the conventional body fluid suction tube is connected to a suction pump, so that the conventional body fluid suction tube can suck in a body fluid such as phlegm and saliva inside the trachea with a suction power of the suction pump.

It should be noted that the conventional tracheal insertion tube is provided with an adaptor at a base portion thereof. Accordingly, it is possible to deal with a change in a posture of the patient. The adaptor is configured to be freely rotatable. Further, a base portion of the conventional body fluid suction tube is supported on the adaptor.

The conventional body fluid suction tube is formed of a flexible material. Accordingly, when the conventional body fluid suction tube is inserted into the conventional tracheal insertion tube, the conventional body fluid suction tube is retained in the conventional tracheal insertion tube such a way that an outer surface of the conventional body fluid suction tube is closely contact with a curved inner surface of the conventional tracheal insertion tube. Further, a distal end portion of the conventional body fluid suction tube protrudes from a distal end portion (an opening portion) of the conventional tracheal insertion tube inside the trachea of the patient. In this state, the conventional body fluid suction tube can suck in the body fluid through the distal end portion thereof (refer to Patent Publication).

### PRIOR ART DOCUMENT

### PATENT PUBLICATION

Japanese Patent Publication No. 2007-307185

### SUMMARY OF THE INVENTION

### PROBLEM TP BE SOLVED BY THE INVENTION

In the conventional body fluid suction tube, it is arranged such that an outer surface of the conventional body fluid suction tube is closely contacted with an inner surface of the conventional tracheal insertion tube inside the conventional tracheal insertion tube. Accordingly, when the patient changes a posture thereof, the conventional body fluid suction tube tends to be rotated relative to the conventional tracheal insertion tube together with the adaptor disposed on the conventional tracheal insertion tube to be freely rotatable. As a result, the distal end portion of the conventional body fluid suction tube may be moved to a different position.

Further, in the conventional body fluid suction tube, the distal end portion of the conventional body fluid suction tube protrudes from the distal end portion (an opening portion) of the conventional tracheal insertion tube inside the trachea of the patient. Accordingly, when the conventional body fluid suction tube sucks in the body fluid through the distal end portion thereof, the conventional body fluid suction tube may apply a sucking force to an inner wall of the trachea of the patient unless the suction power of the suction pump is adequately adjusted.

Further, in the conventional body fluid suction tube, when the distal end portion of the conventional body fluid suction tube is moved, or the sucking force thereof is applied to the inner wall of the trachea of the patient, it may be difficult to smoothly suck in the body fluid, thereby causing discomfort to the patient.

Accordingly, an object of the present invention is to provide a body fluid suction tube capable of solving the problem of the conventional body fluid suction tube. A further object of the present invention is to provide an airway device and a method of using the same.

### MEAN TO SOLVE THE PROBLEM

In order to attain the objects described above, according to the present invention recited in claim 1, an airway device is configured such that a body fluid suction tube is inserted into a tracheal insertion tube to be passed through a trachea of a patient through an adaptor, so that the body fluid suction tube can suck in a body fluid such as phlegm and saliva in the trachea of the patient. The adaptor is disposed at a base end portion of the tracheal insertion tube to be passed through the trachea of the patient, so that the adaptor is freely rotatable.

According to the present invention recited in claim 1, the body fluid suction tube is arranged such that an opening portion thereof at a distal end portion thereof is situated inside relative to an opening portion of the tracheal insertion tube at a distal end portion thereof. Further, the body fluid suction tube is arranged such that only the distal end portion thereof is urged against an inner surface of the tracheal insertion tube at the distal end portion thereof through an elastic force all the time when the adaptor is rotated. Accordingly, a lower side of an outer surface of the body fluid suction tube in a curved shape is separated and is not closely contact with an upper side of the inner surface of the tracheal insertion tube in a curved shape.

According to the present invention recited in claim 2, a body fluid suction tube is inserted into a tracheal insertion tube to be passed through a trachea of a patient through an adaptor, so that the body fluid suction tube can suck in a body fluid such as phlegm and saliva in the trachea of the patient. The body fluid suction tube is arranged such that only a distal end portion thereof is urged against an inner surface of the tracheal insertion tube at the distal end portion thereof through an elastic force when the body fluid suction tube is curved inside the tracheal insertion tube.

According to the present invention recited in claim 3, in the second aspect of the present invention, the body fluid suction tube is in a linear shape in an unused state before the body fluid suction tube is inserted into the tracheal insertion tube.

According to the present invention recited in claim 4, a method of using a body fluid suction tube, in which the body fluid suction tube is inserted into a tracheal insertion tube to be passed through a trachea of a patient through an adaptor, so that the body fluid suction tube can suck in a body fluid such as phlegm and saliva in the trachea of the patient. The body fluid suction tube is arranged such that only a distal end portion thereof is urged against an inner surface of the tracheal insertion tube at the distal end portion thereof through an elastic force when the body fluid suction tube is inserted into and curved inside the tracheal insertion tube.

### EFFECT OF THE INVENTION

According to the present invention, it is possible to achieve the following effects.

According to the present invention, there is provided the body fluid suction tube, in which the body fluid suction tube is inserted into the tracheal insertion tube to be passed through the trachea of the patient, so that the body fluid suction tube can suck in a body fluid such as phlegm and saliva in the trachea of the patient. Further, there is provided the method of using the body fluid suction tube.

According to the present invention, the body fluid suction tube is arranged such that only the distal end portion thereof is urged against the inner surface of the tracheal insertion tube at the distal end portion thereof through an elastic force when the body fluid suction tube is curved inside the tracheal insertion tube. Accordingly, the outer surface of the body fluid suction tube is not closely contact with the inner surface of the tracheal insertion tube. Further, the distal end portion of the body fluid suction tube is not in a state that the distal end portion does protrude from the distal end portion of the tracheal insertion tube. As a result, even when the patient changes a posture thereof, the distal end portion of the body fluid suction tube does not move. Further, it is possible to prevent the body fluid suction tube from applying a sucking force to an inner wall of the trachea of the patient. Therefore, it is possible to smoothly and comfortably suck the body fluid without causing discomfort to the patient.

Further, according to the present invention, the body fluid suction tube is in the linear shape in the unused state before the body fluid suction tube is inserted into the tracheal insertion tube. Accordingly, it is possible to secure an adequate urging force when the body fluid suction tube is curved for use. Further, it is possible to easily store and transport the body fluid suction tube in the unused state.

### BRIEF DESCRIPTION OF THE DRAWINGS

Fig. 1 is a side sectional view showing an operation of a body fluid suction tube according to an embodiment of the present invention; and
Fig. 2 is a side sectional view showing the operation of the body fluid suction tube in a state that an adaptor is rotated according to the embodiment of the present invention.

### PREFERRED EMBODIMENTS TO CARRY OUT THE INVENTION

Hereunder, embodiments of the present invention will be described with regard to an airway device, a body fluid suction tube, and a method of using the body fluid suction tube with reference to the accompanying drawings.

According to the present invention, the body fluid suction tube is inserted into a tracheal insertion tube to be passed through a trachea of a patient, so that the trachea of the patient is secured. The tracheal insertion tube may include a tracheal cannula and an insertion tube. Accordingly, the body fluid suction tube can suck in a body fluid such as phlegm and saliva in the trachea of the patient. The body fluid suction tube may include a suction catheter. In the following description, an airway device, in which the body fluid suction tube (the suction catheter) is inserted into the tracheal insertion tube (the tracheal cannula), will be described as a typical example.

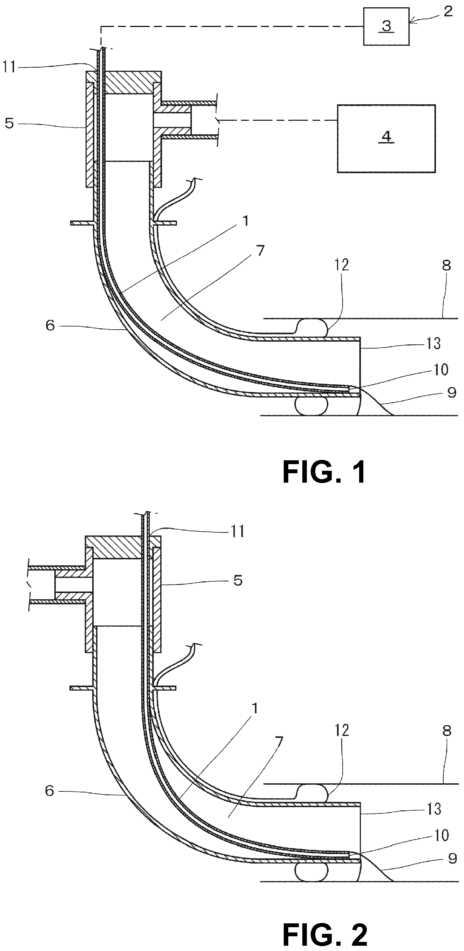

As shown in Fig. 1, the body fluid suction tube 1 is connected to a suction pump 3 of a body fluid suction device 2. Further, the tracheal insertion tube 6 (the tracheal cannula) is connected to an artificial ventilator 4 through an adaptor 5, and the body fluid suction tube 1 is inserted into an air passage 7 of the tracheal insertion tube 6 (the tracheal cannula) curved in a substantially L character shape. Accordingly, the body fluid suction tube 1 can suck in a body fluid 9 such as phlegm and saliva retained in the trachea 8 of the patient through an opening portion 10 of the body fluid suction tube 1 at a distal end thereof.

In the embodiment, the adaptor 5 is attached to a base portion of the tracheal insertion tube 6 to be freely rotatable, so that the body fluid suction tube 1 can be adjustable according to a change in a posture of the patient. Further, the adaptor 5 includes a through hole portion 11, so that a base portion of the body fluid suction tube 1 is held at the through hole portion 11. Further, the tracheal insertion tube 6 is inserted into the trachea 8 of the patient, which is cut open, through a cuff 12 that is freely contracted and expanded. Accordingly, the tracheal insertion tube 6 is arranged in a state that a distal end portion thereof is separated from a surface of the trachea 8 by a distance corresponding to a thickness of the cuff 12.

As described above, in the embodiment, the adaptor 5 is attached to the base portion of the tracheal insertion tube 6 to be freely rotatable. Further, the body fluid suction tube 1 is inserted into the tracheal insertion tube 6 through the adaptor 5, so that the body fluid suction tube 1 can suck in the body fluid 9 such as phlegm and saliva in the trachea 8 of the patient.

In the embodiment, when the body fluid suction tube 1 is used, first, the body fluid suction tube 1 is inserted into the air passage 7 of the tracheal insertion tube 6 through the through hole portion 11 of the tracheal insertion tube 6. More specifically, the body fluid suction tube 1 is inserted into the tracheal insertion tube 6 such that the body fluid suction tube 1 at the distal end portion of the body fluid suction tube 1 is situated just before the opening portion 13 at the distal end portion of the tracheal insertion tube 6 (an inside of the air passage 7 of the tracheal insertion tube 6) and close to the opening portion 13.

In the embodiment, the body fluid suction tube 1 is formed of an elastic material, so that the body fluid suction tube 1 is in a linear shape when the body fluid suction tube 1 is not used. Accordingly, when the body fluid suction tube 1 is inserted into the air passage 7 of the tracheal insertion tube 6 that is curbed in use, the body fluid suction tube 1 is forcibly curved inside the air passage 7. Further, as described above, the body fluid suction tube 1 is formed of an elastic material. Accordingly, when the body fluid suction tube 1 is inserted into the air passage 7 of the tracheal insertion tube 6, an elastic force (a restoration force) is applied to the body fluid suction tube 1 against an elastic force caused by the forcible bending thereof.

Accordingly, in the embodiment, as shown in Fig. 1, the body fluid suction tube 1 is arranged such that only the distal end portion thereof is urged against an inner surface of the air passage 7 of the tracheal insertion tube 6 at the distal end portion thereof through the elastic force when the body fluid suction tube 1 is used. Further, a lower side of an outer surface of the body fluid suction tube 1 is separated (floated) from an upper side of the inner surface of the tracheal insertion tube 6.

In particular, when the body fluid suction tube 1 is used and inserted into the tracheal insertion tube 6, the distal end portion of the body fluid suction tube 1 is contact with the inner circumferential surface of the distal end portion of the tracheal insertion tube 6 that is in a linear shape. Further, the outer side surface of the body fluid suction tube 1 that is in a curved shape is not contact with the inner circumferential surface of the tracheal insertion tube 6 that is in a curved shape. It should be noted that the body fluid suction tube 1 may be formed of a material or in a shape such that the curved portion thereof has an elastic coefficient greater than that of the distal end portion thereof or the base portion thereof in a state that the body fluid suction tube 1 is inserted into the tracheal insertion tube 6.

In the embodiment, when the body fluid suction tube 1 is connected to the body fluid suction device 2, the body fluid suction tube 1 is held with the air passage 7 of the tracheal insertion tube 6 while the body fluid suction tube 1 passes through the air passage 7 of the tracheal insertion tube 6. In this case, the body fluid suction tube 1 is capable of continuously sucking in the body fluid 9 at a relatively small suction rate such as one to three litters per minute. Accordingly, if the distal end portion of the body fluid suction tube 1 is not in a proper posture, it is difficult to smoothly suck in the body fluid 9. More specifically, if the patient changes a posture thereof, the adaptor 5 is rotated relative to the tracheal insertion tube 6 according to the change in the posture of the patient. Accordingly, it the distal end portion of the body fluid suction tube 1 is moved according to the rotation of the adaptor 5, it is difficult to smoothly suck in the body fluid 9.

In the embodiment, as shown in Fig. 1, the body fluid suction tube 1 is arranged such that only the distal end portion thereof is urged against the inner surface of the air passage 7 of the tracheal insertion tube 6 at the distal end portion thereof through the elastic force. Further, the lower side of the outer surface of the body fluid suction tube 1 is separated from and is not contact with the upper side of the inner surface of the tracheal insertion tube 6.

In the embodiment, as shown in Fig. 2, the body fluid suction tube 1 is arranged to adjust a length thereof inside the tracheal insertion tube 6 such that even when the adaptor 5 is rotated according to the change in the posture of the patient, only the distal end portion of the body fluid suction tube 1 is urged against the inner surface of the air passage 7 of the tracheal insertion tube 6 at the distal end portion thereof through the elastic force similar to the arrangement before when the adaptor 5 is rotated. Accordingly, it is possible to maintain the location (the posture) of the opening portion 10 of the body fluid suction tube 1 at the distal end portion thereof.

As described above, in the embodiment, when the body fluid suction tube 1 is inserted into and placed inside the tracheal insertion tube 6 in the curved shape, the body fluid suction tube 1 is arranged such that the opening portion 10 of the body fluid suction tube 1 at the distal end portion thereof is situated at the lower side of the trachea 8 all the time even when the patient changes the posture thereof. When the patient is lying with a face up, the body fluid 9 tends to stay at the lower side of the trachea 8 due to gravity. Accordingly, the body fluid 9 moves toward the tracheal insertion tube 6 along with an air flow of aspiration of the patient, so that the body fluid 9 is sucked in through the opening portion 13 of the tracheal insertion tube 6. In this case, when the opening portion 10 of the body fluid suction tube 1 at the distal end portion thereof is placed at the lower portion on the inner surface of the air passage 7 of the tracheal insertion tube 6 at the distal end portion thereof, the opening portion 10 is situated closer to a location where the body fluid 9 is accumulated. Accordingly, it is possible to smoothly suck in the body fluid 9 with the body fluid suction tube 1.

As described above, in the embodiment, the opening portion 10 of the body fluid suction tube 1 at the distal end portion thereof is situated inside relative to the opening portion 13 of the tracheal insertion tube 6 at the distal end portion thereof. Further, even when the adaptor 5 is rotated, the body fluid suction tube 1 is arranged such that only the distal end portion thereof is urged against the inner surface of the air passage 7 of the tracheal insertion tube 6 at the distal end portion thereof through the elastic force all the time. Further, the lower side of the outer surface of the body fluid suction tube 1 in the curved shape is separated from and is not contact with the upper side of the inner surface of the tracheal insertion tube 6 in the curved shape.

As described above, in the embodiment, when the body fluid suction tube 1 is used and curved inside the tracheal insertion tube 6, the body fluid suction tube 1 is arranged such that only the distal end portion thereof is urged against the inner surface of the air passage 7 of the tracheal insertion tube 6 at the distal end portion thereof through the elastic force.

Accordingly, in the embodiment, the body fluid suction tube 1 is arranged in the tracheal insertion tube 6 such that the outer surface of the body fluid suction tube 1 is not closely contact with the inner surface of the tracheal insertion tube 6. Further, the body fluid suction tube 1 is arranged such that the distal end portion thereof does not protrude from the distal end portion of the tracheal insertion tube 6. Accordingly, even when the patient changes the posture thereof, the distal end portion of the body fluid suction tube 1 is not moved. Further, it is possible to prevent the sucking force from applying to an inner wall of the trachea of the patient. Therefore, it is possible to smoothly and comfortably suck the body fluid without causing discomfort to the patient.

Further, in the embodiment, the body fluid suction tube 1 is in a linear shape in an unused state before the body fluid suction tube 1 is inserted into the tracheal insertion tube 6. Accordingly, it is possible to secure an adequate urging force when the body fluid suction tube 1 is curved for use. Further, it is possible to easily store and transport the body fluid suction tube 1 in the unused state.

## Claims

1. An airway device comprising:
a body fluid suction tube;
a tracheal insertion tube to be passed through a trachea of a patient so that the body fluid suction tube can suck in a body fluid including phlegm and saliva in the trachea of the patient; and
an adaptor disposed at a base end portion of the tracheal insertion tube to be freely rotatable,
wherein said body fluid suction tube is arranged so that an opening portion thereof at a distal end portion thereof is situated inside relative to an opening portion of the tracheal insertion tube at a distal end portion thereof,
said body fluid suction tube is arranged so that only the distal end portion thereof is urged against an inner surface of the tracheal insertion tube at the distal end portion thereof through an elastic force, and
said body fluid suction tube is arranged so that a lower side of an outer surface thereof is separated and is not closely contact with an upper side of the inner surface of the tracheal insertion tube.

2. A body fluid suction tube to be inserted into a tracheal insertion tube to be passed through a trachea of a patient through an adaptor so that the body fluid suction tube can suck in a body fluid including phlegm and saliva in the trachea of the patient,
wherein said body fluid suction tube is arranged so that only a distal end portion thereof is urged against an inner surface of the tracheal insertion tube at a distal end portion thereof through an elastic force when the body fluid suction tube is curved inside the tracheal insertion tube.
body fluid suction tube is in a linear shape in an unused state before the body fluid suction tube is inserted into the tracheal insertion tube.

3. The body fluid suction tube according to claim 2, wherein said body fluid suction tube is in a linear shape in an unused state before the body fluid suction tube is inserted into the tracheal insertion tube.

4. A method of using the body fluid suction tube, comprising the steps of:
inserting the body fluid suction tube into a tracheal insertion tube to be passed through a trachea of a patient through an adaptor so that the body fluid suction tube can suck in a body fluid including phlegm and saliva in the trachea of the patient, and
arranging the body fluid suction tube so that that only a distal end portion thereof is urged against an inner surface of the tracheal insertion tube at a distal end portion thereof through an elastic force when the body fluid suction tube is inserted into and curved inside the tracheal insertion tube.
